# EUROPEAN PATENT APPLICATION

(11) **EP 4 631 491 A1**
(43) Date of publication of application: **15.10.2025**
(21) Application number: 22967969.1
(22) Date of filing: 16.12.2022
(51) Int. Cl.: A61K 8/64, A61K 38/08, C07K 7/06, A61Q 19/00, A61Q 19/02, A61Q 19/08, A61P 17/00

(54) **PEPTIDE HAVING ACTIVITY OF IMPROVING STATE OF SKIN, AND USE THEREOF**

(30) Priority: 07.12.2022 KR 20220170037
(71) Applicant: Caregen Co., Ltd., Anyang-si, Gyeonggi-do 14119 (KR)
(72) Inventor: CHUNG, Yong Ji, Anyang-si, Gyeonggi-do 14119 (KR); KIM, Eun Mi, Anyang-si, Gyeonggi-do 14119 (KR); LEE, Eung Ji, Anyang-si, Gyeonggi-do 14119 (KR); KANG, Hana, Anyang-si, Gyeonggi-do 14119 (KR); HWANG, Bobyeol, Anyang-si, Gyeonggi-do 14119 (KR)
(74) Representative: Potter Clarkson
(86) International application number: PCT/KR2022/020539
(87) International publication number: WO 2024/122713

(57) **Abstract**

The present application relates to a peptide having activity of improving the state of skin, and uses thereof, and provides a peptide consisting of an amino acid sequence of SEQ ID NO: 1, and a cosmetic composition for improving the state of skin, including, as an active ingredient, the peptide consisting of the amino acid sequence of SEQ ID NO: 1.

## Description

### Technical Field

The present application relates to peptides having the activity of improving the state of skin, and uses thereof.

### Background Art

Human skin is constantly subjected to changes, the most representative of which is the deterioration of skin function and decrease in visual beauty due to aging. Aging causes the formation of wrinkles in the skin, and typical factors for wrinkle formation include exposure to ultraviolet rays and decreased collagen biosynthesis, etc. Skin aging may be broadly categorized into endogenous aging, which is caused by genetic factors, and exogenous aging, which is caused by external environmental factors such as sun rays. Among them, it is known that, in the case of exogenous aging, aging may be prevented, treated or delayed by removing reactive oxygen species, promoting fibroblast proliferation and collagen biosynthesis, etc.

On the other hand, collagen, a major component of the extracellular matrix, is the main matrix protein produced by fibroblasts in the skin. Collagen forms the majority of organic matter in skin, tendons, bones, and teeth, with its content being particularly high in bone and skin (dermis). This collagen reduces with age and photoaging caused by ultraviolet irradiation, and this is known to be closely related to the formation of wrinkles in the skin. Furthermore, collagen plays an important role in wound healing, and by stimulating the synthesis of collagen in damaged epithelium, wounds may be repaired quickly and without scarring. In addition, it has been reported that as the biosynthesis of collagen is promoted, the density of the basal layer, etc. increases, the melanin pigment concentration per unit skin density decreases, and the effect of brightening skin tone may be expected.

Meanwhile, pigments that affect skin color are melanin, melanoid, carotene, hemoglobin, and carotenoids, etc., and the various colors of skin, hair, and eyes are determined by these pigments. Among these, the most important pigment in determining skin color is melanin, and specifically, skin color is determined by the amount and distribution of melanin. Melanin is produced in cells called melanocytes beneath the epidermis of the skin, and through skin metabolism, moves to the surface of the stratum corneum and is then shed. The number of melanocytes is almost the same regardless of skin color, but skin color differs simply because the amount, type, and distribution of melanin produced are different.

Against this technical background, multifaceted studies are being conducted to improve the state of skin through mechanisms such as promoting collagen biosynthesis and enhancing the proliferation and activity of fibroblasts. (Korean Registered Patent No. 10-1813629), but these are still insufficient.

### Disclosure of Invention

### Technical Problem

One aspect is to provide a peptide consisting of an amino acid sequence of SEQ ID NO: 1.

Another aspect is to provide a composition for improving the state of the skin, including, as an active ingredient, a peptide consisting of an amino acid sequence of SEQ ID NO: 1.

Another aspect is to provide a pharmaceutical composition for preventing or treating melanin hyperpigmentation disease, including, as an active ingredient, a peptide consisting of an amino acid sequence of SEQ ID NO: 1.

Other objectives and advantages of the present application will become apparent from the following detailed description, together with the appended claims and drawings. Any matter not described herein will be sufficiently recognized and inferred by those skilled in the art of the present application or a similar art, and the description is hereby omitted.

### Solution to Problem

Each of the descriptions and embodiments disclosed in the present application may be applied to other descriptions and embodiments. In other words, all combinations of the various elements disclosed in the present application fall within the scope of the present application. Furthermore, the scope of the present application is not to be considered limited by the specific description set forth below.

One aspect provides a peptide consisting of an amino acid sequence of SEQ ID NO: 1.

As used herein, the term "peptide" may refer to a linear molecule formed by combining amino acid residues to each other by peptide bonds. The peptides may be prepared according to chemical synthesis methods known in the art, in particular solid phase synthesis technique or liquid phase synthesis technique (US Registered Patent No. 5,516,891). As a result of diligent efforts to develop a peptide with biologically effective activity, the inventors of the present disclosure have identified a peptide consisting of an amino acid sequence of SEQ ID NO: 1. Herein, the biologically effective activity may be any one or more selected from the following characteristics: (a) enhancing the expression of extracellular matrix components such as collagen type 1 (Col1a1), fibronectin, elastin, or hyaluronic acid; (b) enhancing the expression of skin barrier factors such as sirtuin-1 (SIRT-1) or aquaporin-3 (AQP3); (c) reducing the level of reactive oxygen species; (d) reducing the expression of matrix metalloprotease-1 (MMP-1); and (e) inhibiting melanin production. Thus, the peptide may be utilized for uses for improving the state of the skin, for skin whitening, or for antioxidant uses.

The peptide may have a protecting group bonded to the N- or C-terminus of the peptide to acquire chemical stability, enhanced pharmacological properties (half-life, absorption, titer, effect, etc.), altered specificity (for example, broad spectrum of biological activity), or reduced antigenicity. In an embodiment, an N-terminus of the peptide may be bonded with any one protecting group selected from the group consisting of an acetyl group, a fluoreonylmethoxycarbonyl group, a formyl group, a palmitoyl group, myristyl group, stearyl group, butoxycarbonyl group, allyloxycarbonyl group, and polyethylene glycol (PEG); and/or the C-terminus of the peptide may be bonded with any one protecting group selected from the group consisting of an amino group(-NH₂), a tertiary alkyl group, and an azide (-NHNH₂). Additionally, the peptide may optionally further include a targeting sequence, a tag, labeled residues, or an amino acid sequence prepared for the specific purpose of increasing the half-life or peptide stability.

The above peptide is artificially synthesized, or non-naturally occurring or engineered, and the "non-naturally occurring or engineered" refers to a state that is not in its natural state, but is created by artificial modification. Here, the artificial modification may include artificially synthesizing an amino acid sequence by mimicking a plurality of amino acid structures, or manipulating it to acquire chemical stability, enhanced pharmacological properties, altered specificity, or reduced antigenicity as described above.

As used herein, the term "stability" may refer to not only in vivo stability, which protects the peptide from attack by proteolytic enzymes in vivo, but also storage stability (for example, room temperature storage stability).

Another aspect provides a cosmetic composition for improving the state of the skin, including, as an active ingredient, a peptide consisting of an amino acid sequence of SEQ ID NO: 1.

The terms or elements mentioned in the description of the peptide that are identical to those already mentioned are as described above.

As used herein, the term "ameliorate" may refer to any act that at least reduces a parameter associated with the alleviation or treatment of a condition, for example the severity of a symptom.

As used herein, the term "improving the state of the skin" may comprehensively refer to the process or effect of treating, reducing, or alleviating skin damage, etc., caused by endogenous or exogenous factors of the skin, such as for example, the term may be interpreted as showing improvement of wrinkles, improvement of skin wound recovery, strengthening of skin barrier, or inhibition of skin aging, but is not limited thereto.

As used herein, "improving wrinkles," "improving skin elasticity," and "wound recovery" may refer to all actions that increases the total amount of extracellular matrix factors, including, promotion of collagen synthesis, etc. In addition, "strengthening of skin barrier" may also refer to strengthening the skin's natural function to prevent leakage of moisture and nutrients from the skin to the outside, and to prevent the penetration of harmful substances into the skin, such as bacteria and virus, etc. Furthermore, "inhibition of skin aging" may refer to inhibiting the deterioration of skin functions such as wrinkles, sagging of the skin, decreased elasticity, etc. In this case, the skin aging may be photoaging, for example, skin aging caused by ultraviolet rays.

Herein, "skin whitening" may be interpreted as not only brightening the skin tone by inhibiting the synthesis of melanin pigment, but also improving hyperpigmentation of the skin caused by ultraviolet rays, hormones, or genetic factors. Herein, the hyperpigmentation of the skin may include, but is not limited to, freckles, melasma, senile lentigines, brown spots, or age spots, etc.

Although prior functional peptides have effective biological activity, they have shown disadvantages such as not being able to effectively enter target tissue or cell due to the size of the peptide itself, or disappearing from the body in a short period of time due to their short half-life. In contrast, the cosmetic composition according to an example includes, as an active ingredient, a peptide consisting of 10 or fewer amino acids, and accordingly, the penetration rate, etc., of the active ingredient into the skin is very excellent, and the state of the skin may be effectively improved, for example, when applied topically to the skin.

According to an embodiment, the synthesis of extracellular matrix constituent factor and skin barrier factors was able to be enhanced. In addition, the peptide was able to restore the inhibited activity of fibroblasts and keratinocytes and enhance their antioxidant efficacy, as well as significantly inhibit the production of melanin. Thus, the peptide may be utilized as an active ingredient in a cosmetic composition for improving the state of the skin.

The cosmetic composition may include, a cosmetically effective amount of the peptide; and/or a cosmetically acceptable carrier, but is not limited thereto.

As used herein, the term "cosmetically effective amount" refers to an amount sufficient to achieve the state of the skin improvement effect of the cosmetic composition.

The weight ratio between the peptide and a cosmetically acceptable carrier may be, for example, 500:1 to 1:500, for instance, the weight ratio may be 450:1 to 1:450, 400:1 to 1:400, 350:1 to 1:350, 300:1 to 1:300, 250:1 to 1:250, 200: 1 to 1:200, 150:1 to 1:150, 100:1 to 1:100, 80:1 to 1:80, 60:1 to 1:60, 40:1 to 1:40, 20:1 to 1:20, 10:1 to 1:10, 8:1 to 1:8, 6:1 to 1:6, 4:1 to 1:4, or 2:1 to 1:2, but is not limited thereto.

The cosmetic composition may be prepared in any formulation commonly prepared in the art, for example, may be formulated as a solution, a suspension, an emulsion, a paste, a gel, a cream, a lotion, a powder, a soap, a surfactant-containing cleanser, an oil, a powder foundation, an emulsion foundation, a wax foundation, and a spray, etc., but is not limited thereto. For example, the cosmetic composition may be prepared in the form of a softening lotion, nutrition lotion, nutrition cream, massage cream, essence, eye cream, cleansing cream, cleansing foam, cleansing water, mask, spray or powder.

If the formulation of the cosmetic composition is a paste, cream or gel, the carrier component may be an animal oil, vegetable oil, wax, paraffin, starch, tragacanth, cellulose derivative, polyethylene glycol, silicone, bentonite, silica, talc or zinc oxide, etc.

If the formulation of the cosmetic composition is a powder or spray, lactose, talc, silica, aluminum hydroxide, calcium silicate or polyamide powder may be used as a carrier component and, for example, if the formulation is a spray, it may additionally include a propellant such as chlorofluorohydrocarbon, propane/butane or dimethyl ether.

If the formulation of the cosmetic composition is a solution or emulsion, a solvent, solubilizer or emulsifier is used as a carrier component and may include, for example, water, ethanol, isopropanol, ethyl carbonate, ethyl acetate, benzyl alcohol, benzyl benzoate, propylene glycol, 1,3-butyl glycol oil, glycerol aliphatic esters, polyethylene glycol or fatty acid esters of sorbitan.

If the formulation of the cosmetic composition is a suspension, the carrier component may include a liquid diluent such as water, ethanol or propylene glycol, a suspending agent such as ethoxylated isostearyl alcohol, polyoxyethylene sorbitol esters and polyoxyethylene sorbitan esters, microcrystalline cellulose, aluminum methahydroxide, bentonite, agar or tragacanth, etc.

If the formulation of the cosmetic composition is a surfactant-containing cleanser, the carrier component may include aliphatic alcohol sulfate, aliphatic alcohol ether sulfate, sulfosuccinic acid monoester, isethionate, imidazolinium derivative, methyl taurate, sarcosinate, fatty acid amide ether sulfate, alkylamidobetaine, aliphatic alcohol, fatty acid glyceride, fatty acid diethanolamide, vegetable oil, lanolin derivative, or ethoxylated glycerol fatty acid ester, etc.

The peptide may be incorporated into a nanosome or a nanoparticle to further improve skin penetration or stability issues. For example, the nanosome may be prepared by a microfluidizer using lecithin as a raw material, and may be incorporated in lecithin particles. Any method of preparing the nanosome may be used as long as it is known. The size of the nanosome particle is preferably 30 to 200 nm. **If** the size of the nanosome particle is less than 30 nm, skin penetration may progress very quickly, resulting in skin side effects, and if the size is greater than 200 nm, skin penetration may not be easy, making it difficult to achieve the effect of using the nanosome structure.

The ingredients included in the cosmetic composition include, in addition to peptides and carrier components as active ingredients, components commonly used in cosmetic compositions, and may include, for example, common adjuvants such as an antioxidant, stabilizer, solubilizer, vitamin, pigment, and fragrance.

The content of the peptide as an active ingredient contained in the cosmetic composition may be selected appropriately without limitation depending on the form of the product, the desired use, etc., and may be added, for example, from 0.01 to 15 wt% of the total weight of the cosmetic composition. Additionally, for example, the cosmetic composition may include the peptide in an amount of from 1.0 wt% to 3.0 wt%, preferably from 2.0 wt% to 3.0 wt%, based on the total weight.

Another aspect provides a method of improving the state of the skin, including applying to the skin of an individual a cosmetic composition including, as an active ingredient, a peptide consisting of an amino acid sequence of SEQ ID NO: 1; and a use of a peptide consisting of an amino acid sequence of SEQ ID NO: 1 for the preparation of a composition for improving the state of the skin.

In the above description of the cosmetic composition, the terms or elements referred to are the same as those already mentioned.

As used herein, the term "subject" refers to a subject in need of improvement of the state of the skin, and more specifically, a mammal, such as a human or non-human primate, mouse, dog, cat, horse, and bovine, etc.

As used herein, the terms "applying," "administering," and "applying" are used interchangeably and may refer to causing at least partial localization of a composition according to an example to a desired site, or to placing a composition according to an example into an individual by route of administration.

Another aspect provides a pharmaceutical composition for preventing or treating melanin hyperpigmentation disease, including, as an active ingredient, a peptide consisting of an amino acid sequence of SEQ ID NO: 1.

In the above description of the peptide, the terms or elements referred to are the same as those already mentioned.

As used herein, the term "prevention" refers to any act of inhibiting or delaying the onset of a disease by administering the composition.

As used herein, the term "treatment" refers to any form of treatment that provides an effect to a subject suffering from a disease or at risk of developing a disease, including improving the condition of the subject (for example, one or more symptoms), delaying the progression of the disease, delaying the onset of symptoms, or slowing the progression of symptoms, etc. Therefore, the above "treatment" and "prevention" are not intended to refer to cure or complete elimination of symptoms.

The "melanin hyperpigmentation disease", which is a disease to be prevented or treated by the pharmaceutical composition above, may be a general term for diseases in which melanin pigment is excessively produced and deposited on the skin, and may be, for example, melasma, freckle, senile pigmentation or solar lentigines, but is not limited thereto.

The pharmaceutical composition may include, a pharmaceutically effective amount of the peptide; and/or a pharmaceutically acceptable carrier, but is not limited thereto.

As used herein, the term "pharmaceutically effective amount" may refer to an amount sufficient to achieve the efficacy of the pharmaceutical composition for preventing or treating melanin hyperpigmentation disease.

The pharmaceutically acceptable carrier is commonly used in formulations and include, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil, etc., but is not limited thereto. Suitable pharmaceutically acceptable carriers and formulations are described in detail in Remington's Pharmaceutical Sciences (19th ed., 1995).

The weight ratio between the peptide and a pharmaceutically acceptable carrier may be, for example, 500:1 to 1:500, for instance, the weight ratio may be 450:1 to 1:450, 400:1 to 1:400, 350:1 to 1:350, 300:1 to 1:300, 250:1 to 1:250, 200: 1 to 1:200, 150:1 to 1:150, 100:1 to 1:100, 80:1 to 1:80, 60:1 to 1:60, 40:1 to 1:40, 20:1 to 1:20, 10:1 to 1:10, 8:1 to 1:8, 6:1 to 1:6, 4:1 to 1:4, or 2:1 to 1:2, but is not limited thereto.

In addition to the above ingredients, the pharmaceutical composition may additionally include, lubricants, humectant, sweetener, flavoring agent, emulsifier, suspending agent, preservative, etc., but is not limited thereto.

The pharmaceutical composition may be administered orally or parenterally, preferably parenterally, and if parenterally administered, may be administered by, but not limited to, intramuscular injection, intravenous injection, subcutaneous injection, intraperitoneal injection, topical administration, transdermal administration, etc.

The dosage of the pharmaceutical composition may be, but is not limited to, 0.0001 to 1000 ug (micrograms), 0.001 to 1000 ug, 0.01 to 1000 ug, 0.1 to 1000 ug, or 1.0 to 1000 ug per day, and may be varied by factors such as method of formulation, mode of administration, patient age, weight, sex, medical condition, food, time of administration, route of administration, rate of excretion, and response sensitivity.

The pharmaceutical composition may be formulated, with a pharmaceutically acceptable carrier and/or excipient, according to a method that could be readily practiced by a person skilled in the art to which the present disclosure belongs, and the composition may be prepared into a unit dosage form or may be contained in a multi-dose container.

The formulation may be in the form of a solution, suspension or emulsion in an oil or aqueous medium, or in the form of an extract, powder, granule, tablet or capsule, and may additionally include a dispersant and/or stabilizer.

Another aspect provides a method of preventing or treating a melanin hyperpigmentation disease, including administering to a subject a pharmaceutical composition including, as an active ingredient, a therapeutically effective amount of a peptide consisting of an amino acid sequence of SEQ ID NO: 1.

In the above description of the pharmaceutical composition, the terms or elements referred to are the same as those already mentioned.

Another aspect provides an antioxidant composition including, as an active ingredient, a peptide consisting of an amino acid sequence of SEQ ID NO: 1.

In the above description of the peptide, the terms or elements referred to are the same as those already mentioned.

The antioxidant composition may be in the form of a pharmaceutical composition, a prodrug composition, or a cosmetic composition, and in an example, the composition may be utilized as a cosmetic composition for improving the state of the skin, or as a pharmaceutical composition for improving or treating the condition of a disease associated with skin damage.

According to an example, the peptide was shown to be able to restore the inhibited activity of fibroblasts and keratinocytes and enhance their antioxidant effect. Thus, the peptide may be utilized as an active ingredient in an antioxidant composition.

The antioxidant composition may be provided, for example, in the form of a pharmaceutical composition. The pharmaceutical composition may include, a pharmaceutically effective amount of the peptide; and/or a pharmaceutically acceptable carrier. In the above description of the pharmaceutical composition, the terms or elements referred to are the same as those already mentioned.

The antioxidant composition may be provided, for example, in the form of a pharmaceutical composition. The pharmaceutical composition may include, a pharmaceutically effective amount of the peptide; and/or a pharmaceutically acceptable carrier. The terms or elements mentioned in the description of the pharmaceutical composition that are identical to those already mentioned are as described above.

Another aspect provides a food composition for improving the state of the skin including, as an active ingredient, a peptide consisting of an amino acid sequence of SEQ ID NO: 1.

The terms or elements mentioned in the description of the peptide that are identical to those already mentioned are as described above.

The content of the peptide as an active ingredient contained in the food composition may be appropriately selected without limitation depending on the form of the food, the desired use, etc., and may be added, for example, from 0.01 to 15 wt% of the total weight of the food. Additionally, for example, the health drink composition may be added at a rate of 0.02 to 10 g, preferably 0.3 to 1 g, based on 100 ml.

### Advantageous Effects of Invention

According to a peptide according to an aspect, it may be applied to improve the state of the skins including wrinkle improvement, skin elasticity improvement, wound healing, skin barrier strengthening, skin aging inhibition, or skin whitening, etc., by promoting the synthesis of extracellular matrix components and skin barrier factors.

According to a peptide according to an aspect, it may contribute to repairing skin damage that may be caused by the external environment, such as ultraviolet rays, etc., by restoring the impaired activity of fibroblasts and keratinocytes and enhancing their antioxidant capacity.

Therefore, the peptide according to an aspect may be utilized as an active ingredient of a composition for improving the state of the skin or a pharmaceutical composition for preventing or treating melanin hyperpigmentation disease.

### Brief Description of Drawings

FIG. 1 shows the results confirming increases in the expression of extracellular matrix components of Col1a1, fibronectin, and elastin after adding a peptide consisting of an amino acid sequence of SEQ ID NO: 1 to NIH3T3 cells.
FIG. 2 shows a quantitative evaluation of the expression levels of extracellular matrix components after adding the peptide consisting of the amino acid sequence of SEQ ID NO: 1 to NIH3T3 cells, wherein FIG. 2A shows the results of confirming the expression levels of Col1a1, FIG. 2B shows results of confirming the expression levels of fibronectin, and FIG. 2C shows the results of confirming the expression levels of elastin.
FIG. 3 shows the results of quantitatively evaluating the expression levels of procollagen I alpha after adding the peptide consisting of the amino acid sequence of SEQ ID NO: 1 to NIH3T3 cells.
FIG. 4 shows the results of quantitatively evaluating the expression levels of hyaluronic acid after adding the peptide consisting of the amino acid sequence of SEQ ID NO: 1 to HaCaT cells.
FIG. 5 shows the results of confirming the expression levels of fibronectin and elastin, which are extracellular matrix components, after adding the peptide consisting of the amino acid sequence of SEQ ID NO: 1 and a comparison group peptide to NIH3T3 cells.
FIG. 6 shows a quantitative evaluation of the expression levels of extracellular matrix components after adding the peptide consisting of the amino acid sequence of SEQ ID NO: 1 and a comparison group peptide to NIH3T3 cells, wherein FIG. 6A shows the results of confirming the expression levels of fibronectin, and FIG. 6B shows the results of confirming the expression levels of elastin.

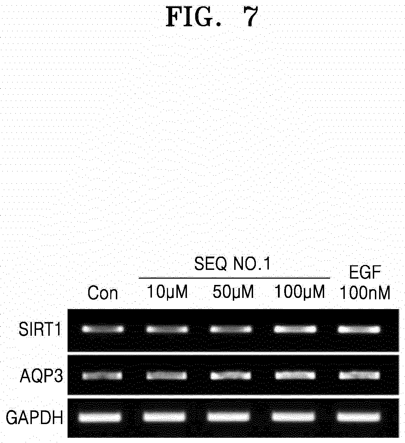

FIG. 7 shows the results of confirming increases in the expression of skin barrier factors SIRT-1 and AQP3 after adding the peptide consisting of the amino acid sequence of SEQ ID NO: 1 to HaCaT cells.

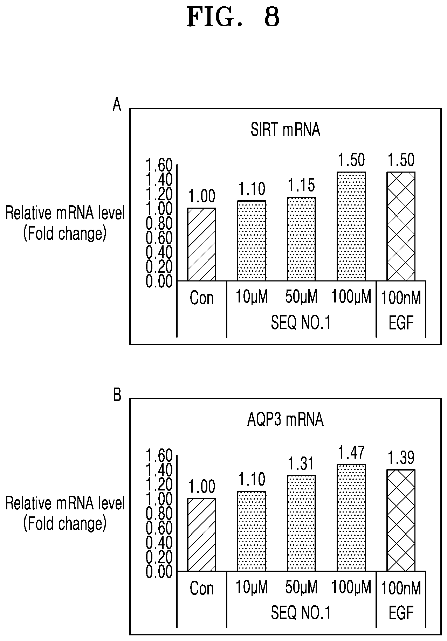

FIG. 8 shows a quantitative evaluation of the expression levels of skin barrier factors after adding the peptide consisting of the amino acid sequence of SEQ ID NO: 1 to HaCaT cells, wherein FIG. 8A shows the results of confirming the expression levels of SIRT-1, and FIG. 8B shows the results of confirming the expression levels of AQP3.
FIG. 9 shows the results of quantitatively confirming changes in the levels of reactive oxygen species increased by ultraviolet rays, after adding the peptide consisting of the amino acid sequence of SEQ ID NO: 1 to NIH3T3 cells.
FIG. 10 shows the results of quantitatively confirming changes in the levels of reactive oxygen species increased by ultraviolet rays, after adding the peptide consisting of the amino acid sequence of SEQ ID NO: 1 to HaCaT cells.
FIG. 11 shows the results of quantitatively confirming changes in the levels of reactive oxygen species increased by ultraviolet rays, after adding the peptide consisting of the amino acid sequence of SEQ ID NO: 1 and a comparison group peptide to HaCaT cells.
FIG. 12 shows the results of confirming changes in the expression levels of SIRT-1 and AQP3 reduced by ultraviolet rays, after adding the peptide consisting of the amino acid sequence of SEQ ID NO: 1 to HaCaT cells.
FIG. 13 shows a quantitative confirmation of changes in expression levels of SIRT-1 and AQP3 reduced by ultraviolet rays, after adding the peptide consisting of the amino acid sequence of SEQ ID NO: 1 to HaCaT cells, wherein FIG. 13A shows the results of confirming the expression levels of SIRT-1, and FIG. 13B shows the results of confirming the expression levels of AQP3.
FIG. 14 shows the results of confirming changes in the expression levels of MMP-1 increased by ultraviolet rays, after adding the peptide consisting of the amino acid sequence of SEQ ID NO: 1 to NIH3T3 cells.
FIG. 15 shows the results of confirming changes in the expression levels of MMP-1 increased by ultraviolet rays, after adding the peptide consisting of the amino acid sequence of SEQ ID NO: 1 to HaCaT cells.
FIG. 16 shows the results of quantitatively confirming changes in levels of melanin production after adding the peptide consisting of the amino acid sequence of SEQ ID NO: 1 and a comparison group peptide to B16F10 cells.

### Mode for the Invention

Hereinafter, the present disclosure will be described in more detail with reference to examples. However, these examples are intended to illustrate the present disclosure by way of example and the scope of the present disclosure is not limited to these examples.

### Example 1. Synthesis of peptides

A peptide with an amino acid sequence of SEQ ID NO: 1 listed in Table 1 below was synthesized using an automated peptide synthesizer (Milligen 9050, Millipore, USA), and the synthesized peptide was purified by C18 reversed-phase high performance liquid chromatography (HPLC) (Waters Associates, USA). The column used was an ACQUITY UPLC BEH300 C18 (2.1 mm X 100 mm, 1.7 µm, Waters Co, USA).

**[Table 1]**

| **SEQ ID NO:** | **Sequence (N terminus -> C terminus)** |
|---|---|
| 1 | GPRGDP |

### Example 2. Confirmation of improvement in wrinkles, improvement in skin elasticity

### 2-1. Efficacy evaluation of peptides according to an embodiment

In this example, the aim was to confirm the effect of the peptide according to an example on improving endogenous skin aging, including improvement of wrinkles and enhancement of elasticity, etc., by evaluating the expression levels of collagen type 1 (Col1a1), fibronectin, elastin, and hyaluronic acid, which are known components of the dermis.

Specifically, NIH3T3 cells were seeded in a 6-well plate at a density of 3×10⁵ cells/well and cultured for 24 hours. Next, the cells were washed once with serum-free DMEM media, and 10 µM, 50 µM or 1000 µM of a peptide consisting of an amino acid sequence of SEQ ID NO: 1 was dispensed into 1 mL of the serum-free media, and cultured in a CO₂ incubator at 37 °C for 24 hours. Next, the culture was washed twice with PBS, and RNA was isolated from the culture using easy blue (iNtRON, Cat. No.: 17061, Korea). Next, using an RT kit (Enzynomics, Cat. NO.: RT200, Korea), the isolated RNA was reverse transcribed to synthesize the respective cDNA. Next, using the synthesized 1µg cDNA, primers for Col1a1, fibronectin, or elastin, and a PCR kit (Enzynomics, Cat. No.: P581T, Korea), polymerase chain reaction (PCR) was performed. Meanwhile, in this example, an untreated group was used as a control group, and a group to which TGF-β1 was added was used as a positive control group, the nucleotide sequences of the primers used in this example are shown in Table 2 below.

**[Table 2]**

| **Primer Name** | | **Sequence (5'->3')** | **SEQ ID NO:** |
|---|---|---|---|
| Col1a1 | Forward | CACCCTCAAGAGCCTGAGTC | 5 |
| | Reverse | AGACGGCTGAGTAGGGAACA | 6 |
| Fibronectin | Forward | CCAGGAACCGAGTACACCAT | 7 |
| | Reverse | ATACCCAGGTTGGGTGATGA | 8 |
| Elastin | Forward | GCAAGACCTGGCTTTGGACT | 9 |
| | Reverse | GGGAGTTTCTGGTTAGGGCTG | 10 |
| GAPDH | Forward | GGAGCCAAAAGGGTCATCAT | 11 |
| | Reverse | GTGATGGCATGGACTGTGGT | 12 |

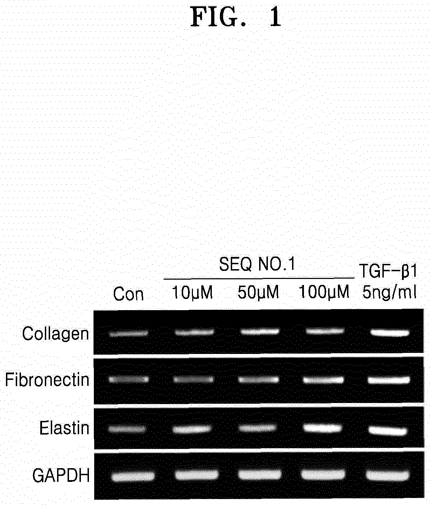

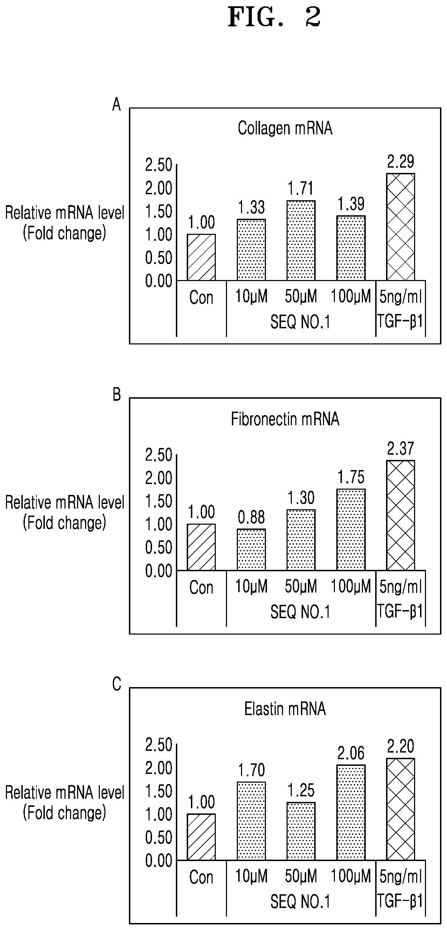

As a result, as shown in FIG. 1 and FIG. 2, it was confirmed that expression of extracellular matrix components Col1a1, fibronectin, and elastin was increased by the peptide consisting of the amino acid sequence of SEQ ID NO: 1.

Additionally, to evaluate the expression levels of procollagen I alpha and hyaluronic acid at the protein level, NIH3T3 cells or HaCaT cells were seeded at a density of 1 × 10⁴ cells/well in 24-well plates and then cultured for 24 hours. Next, the cells were washed once with serum-free DMEM media, and 10 µM, 50 µM or 1000 µM of the peptide consisting of the amino acid sequence of SEQ ID NO: 1 was dispensed into 2 mL of the serum-free media, and cultured in a CO₂ incubator at 37 °C for 72 hours. Next, the culture was centrifuged to settle the suspended matter, and the supernatant was transferred to an e-tube. Next, Collagen ELISA (abcam, Cat No.: ab210579, UK) and HA ELISA (ECHELON biosciences, Cat No.: K-1200, USA) were performed on the centrifuged culture. Meanwhile, in this example, an untreated group was used as the control group, and a group to which TGF-β1 or EGF was added was used as the positive control group.

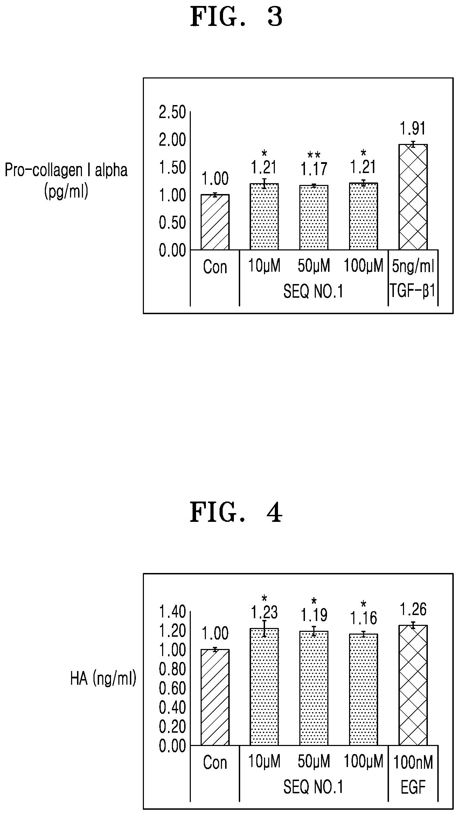

As a result, as shown in FIG. 3 and FIG. 4, it was confirmed that the expression of procollagen I alpha, a precursor of collagen, and hyaluronic acid, which is associated with skin elasticity and skin moisture retention, increased at the protein level by the peptide consisting of the amino acid sequence of SEQ ID NO: 1. Through the above results, it was found that the peptide according to an example contributes to the improvement of endogenous skin aging, including improvement of wrinkles and enhancement of elasticity, etc., by increasing extracellular matrix components.

### 2-2. Comparison of efficacy between peptides and comparison groups according to an embodiment

In this example, the aim was to more specifically compare the effects of peptides according to an embodiment on improving intrinsic skin aging. To this end, a peptide consisting of an amino acid sequence of SEQ ID NO: 2, 3, or 4 having a sequence similar to a peptide according to an embodiment was set as a comparison group, and the expression levels of fibronectin and elastin were evaluated and compared in the same manner as in Example 2-1. Meanwhile, in this example, an untreated group was used as the control group, a group to which the peptide consisting of the amino acid sequence of SEQ ID NO: 2, 3, or 4 was added (50 µM, 100 µM) was used as a comparison group, and a group to which TGF-β1 or EGF was added was used as the positive control group, the nucleotide sequences of the primers used in this example are as shown in Table 2 above.

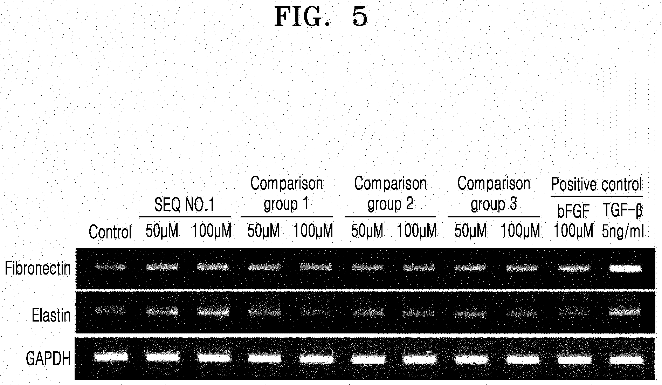

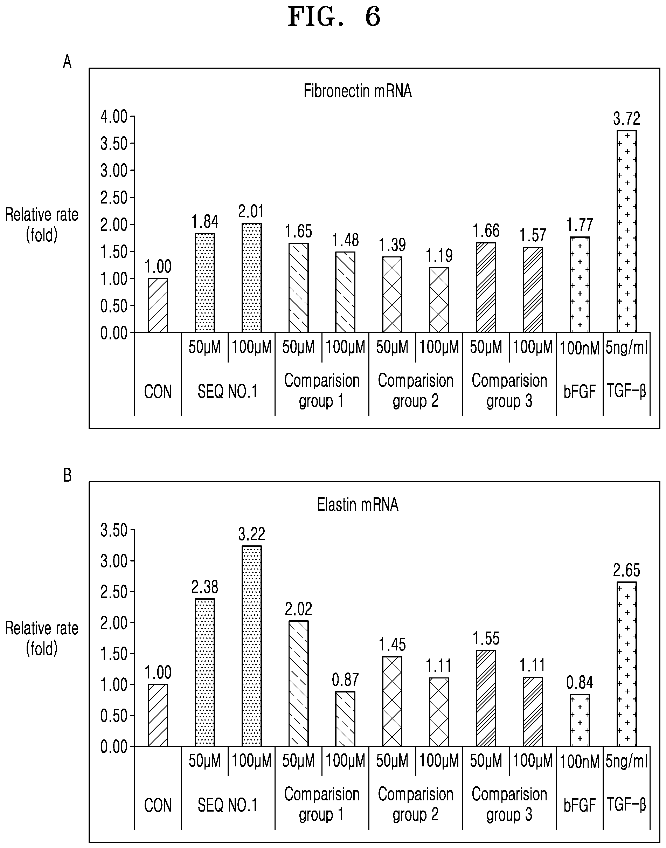

As a result, as shown in FIG. 5 and FIG. 6, expression of fibronectin and elastin, which are extracellular matrix components, was increased by the peptide consisting of the amino acid sequence of SEQ ID NO: 1, and this effect was superior compared to the expression level of the comparison group consisting of a sequence similar to the peptide according to an embodiment.

### Example 3. Confirmation of skin barrier strengthening effect

In this example, the aim was to confirm the effect of the peptides according to an example on strengthening the skin barrier, by evaluating the expression levels of sirtuin-1 (SIRT-1) or aquaporin-3 (AQP3).

Specifically, HaCaT cells were seeded in a 6-well plate at a density of 3×10⁵ cells/well and cultured for 24 hours. Next, the cells were washed once with serum-free DMEM media, and 10 µM, 50 µM or 1000 µM of the peptide consisting of the amino acid sequence of SEQ ID NO: 1 was dispensed into 1 mL of the serum-free media, and cultured in a CO₂ incubator at 37 °C for 24 hours. Next, the culture was washed twice with PBS, and RNA was isolated from the culture using easy blue (iNtRON, Cat. No.: 17061, Korea). Next, using an RT kit (Enzynomics, Cat. NO.: RT200, Korea), the isolated RNA was reverse transcribed to synthesize the respective cDNA. Next, using the synthesized 1µg cDNA, primers for SIRT-1 or AQP3, and a PCR kit (Enzynomics, Cat. No.: P581T, Korea), polymerase chain reaction (PCR) was performed. Meanwhile, in this example, an untreated group was used as the control group, and a group to which EGF was added was used as a positive control group, the nucleotide sequences of the primers used in this example are shown in Table 3 below.

**[Table 3]**

| **Primer Name** | | **Sequence (5'->3')** | **SEQ ID NO:** |
|---|---|---|---|
| SIRT1 | Forward | TCAGTGGCTGGAACAGTGAG | 13 |
| | Reverse | TCTGGCATGTCCCACTATCA | 14 |
| AQP3 | Forward | CCTTCTTGGGTGCTGGAATA | 15 |
| | Reverse | ACACGATAAGGGAGGCTGTG | 16 |
| GAPDH | Forward | GGAGCCAAAAGGGTCATCAT | 11 |
| | Reverse | GTGATGGCATGGACTGTGGT | 12 |

### Example 4. Confirmation of effect of reducing reactive oxygen species increased by ultraviolet rays

### 4-1. Efficacy evaluation of peptides according to an embodiment

In this example, the aim was to confirm the effect of the peptide according to an embodiment on the antioxidant effect in damaged skin, by evaluating the change in the level of reactive oxygen species in skin cells increased by ultraviolet irradiation.

Specifically, NIH3T3 cells or HaCaT cells were seeded in a 6-well plate at a density of 5×10⁵ cells/well and cultured for 24 hours. Next, the cells were washed once with serum-free DMEM media, and 10 µM, 50 µM or 1000 µM of the peptide consisting of the amino acid sequence of SEQ ID NO: 1 was dispensed into 1 mL of the serum-free media, and cultured in a CO₂ incubator at 37 °C for 1 hour. Next, the above culture was transferred to an e-tube, mixed with 1 ml of PBS, and dispensed into a well plate. Next, using a UV irradiator (VILBER LOURMAT, Cat No.: 3102-BSU, France), 6 J/cm² ultraviolet rays were irradiated to NIH3T3 cells, and 15 mJ/cm² ultraviolet rays were irradiated to HaCaT cells. Next, the PBS in the well plate was removed, 900 µL of the culture medium in which the peptides were dispensed was added, and the plate was cultured in a CO₂ incubator at 37 °C for 24 hours. The culture was then treated with DCFG-DA (2',7'-dichlorofluorescin diacetate), wrapped in foil, and cultured in a CO₂ incubator at 37 °C for 30 minutes. Next, the culture was washed twice with PBS and treated with 500 µL of 1× trypsin/EDTA to acquired cells, which were then centrifuged. After washing the centrifuged cells with PBS, FL1 fluorescence values were measured through flow cytometry (FACS, BD, USA). In this example, an untreated group was used as the control group, a group irradiated with only ultraviolet rays was used as a negative control group, and a group to which Nac after ultraviolet irradiation was added was used as the positive control group.

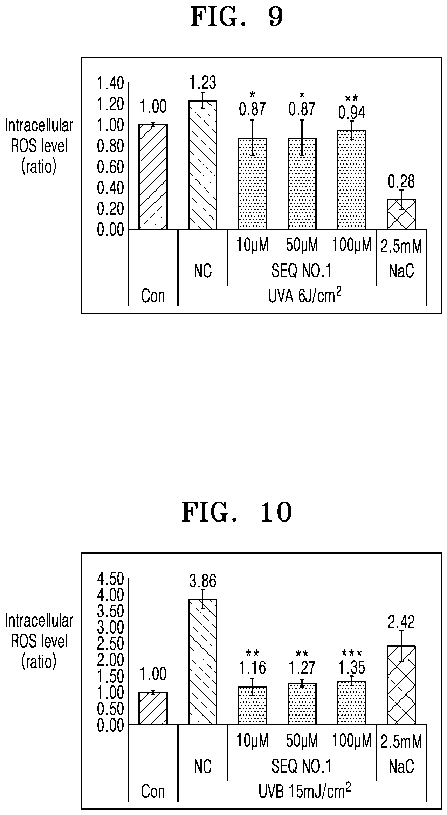

As a result, as shown in FIG. 9 and FIG. 10, it was confirmed that the level of reactive oxygen species in fibroblasts and keratinocytes increased by ultraviolet irradiation was reduced by the peptide consisting of the amino acid sequence of SEQ ID NO: 1. Through the above results, it was found that the peptide according to an example contributes to the antioxidant effect on skin cells induced by ultraviolet rays.

### 4-2. Comparison of efficacy between peptides and comparison groups according to an embodiment

In this example, the aim was to compare in more detail, the effect of peptides according to an embodiment on the antioxidant effect in damaged skin cells. To this end, the peptide consisting of the amino acid sequence of SEQ ID NO: 2, 3, or 4 having a sequence similar to the peptide according to an embodiment was set as a comparison group, and the level of reactive oxygen species in skin cells increased by ultraviolet irradiation of HaCaT cells was evaluated and compared in the same manner as in Example 4-1. Meanwhile, in this example, an untreated group was used as the control group, a group irradiated only with ultraviolet rays was used as the negative control group, and a group to which the peptide consisting of the amino acid sequence of SEQ ID NO: 2, 3, or 4 was added (50 µM, 100 µM) after ultraviolet irradiation was used as the comparison group, and the positive control group was a group to which Trolox was added after ultraviolet irradiation.

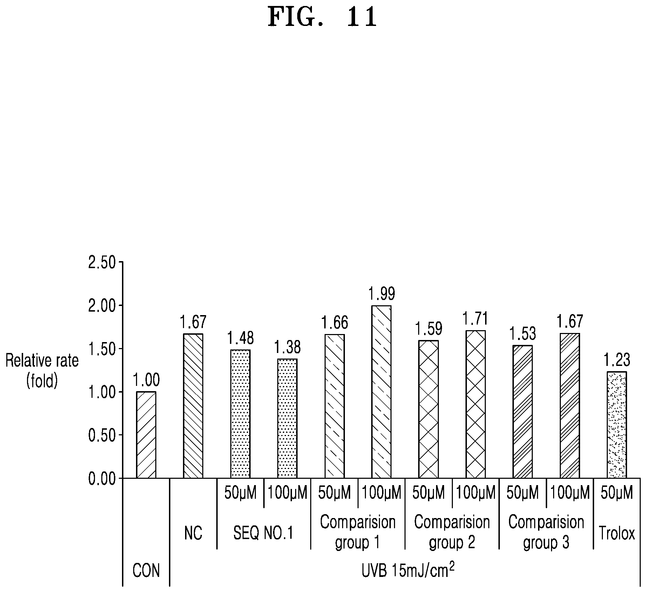

As a result, as shown in Fig. 11, the level of reactive oxygen species in keratinocytes increased by ultraviolet irradiation was reduced by the peptide consisting of the amino acid sequence of SEQ ID NO: 1, and this effect was superior compared to the expression level of the comparison group consisting of a sequence similar to the peptide according to an embodiment.

### Example 5. Confirmation of recovery effect of skin barrier factor reduced by ultraviolet rays

In this example, the aim was to confirm the effect of the peptide according to an embodiment on the recovery of skin barrier factors in damaged skin cells by evaluating changes in the levels of skin barrier factors in skin cells reduced by ultraviolet irradiation.

Specifically, HaCaT cells were seeded in a 6-well plate at a density of 5×10⁵ cells/well and cultured for 24 hours. Next, the cells were washed once with serum-free DMEM media, and 10 µM, 50 µM or 1000 µM of the peptide consisting of the amino acid sequence of SEQ ID NO: 1 was dispensed into 1 mL of the serum-free media, and cultured in a CO₂ incubator at 37 °C for 1 hour. Next, the above culture was transferred to an e-tube, mixed with 1 ml of PBS, and dispensed into a well plate. Next, using a UV irradiator (VILBER LOURMAT, Cat No.: 3102-BSU, France), 15 mJ/cm² ultraviolet rays were irradiated to the HaCaT cells. Next, the PBS in the well plate was removed, 900 µL of the culture medium in which the peptides were dispensed was added, and the plate was cultured in a CO₂ incubator at 37 °C for 8 hours. Next, the culture was washed twice with PBS, and RNA was isolated from the culture using easy blue (iNtRON, Cat. No.: 17061, Korea). Next, using an RT kit (Enzynomics, Cat. NO.: RT200, Korea), the isolated RNA was reverse transcribed to synthesize the respective cDNA. Next, using the synthesized 1µg cDNA, primers for SIRT-1 or AQP3, and a PCR kit (Enzynomics, Cat. No.: P581T, Korea), polymerase chain reaction (PCR) was performed. Meanwhile, in this example, an untreated group was used as the control group, a group irradiated only with ultraviolet rays was used as the comparison group, and a group to which NaC was added was used as the positive control group, the nucleotide sequences of the primers used in this example are as shown in Table 3 above.

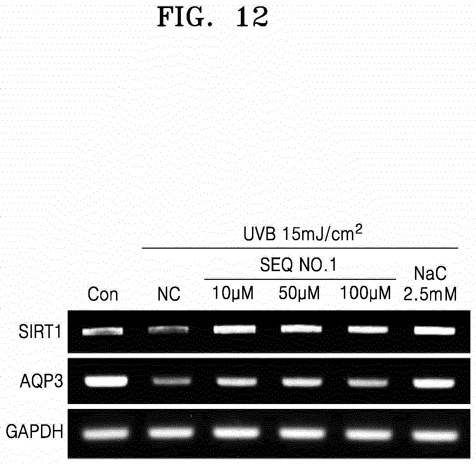

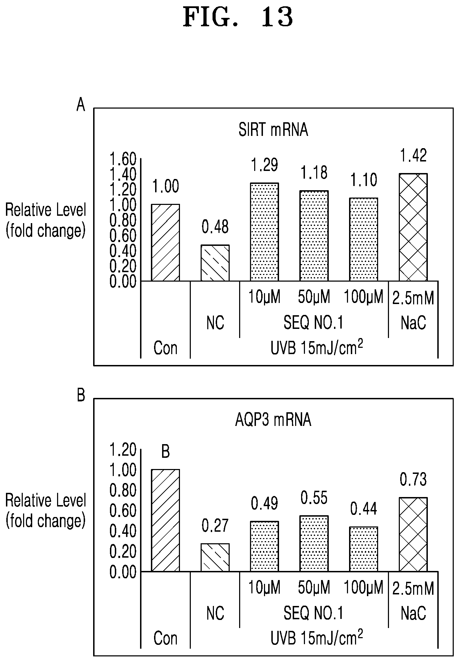

As a result, as shown in FIG. 12 and FIG. 13, it was confirmed that the expression of SIRT-1 and AQP3, reduced by ultraviolet irradiation, was increased or restored by the peptide consisting of the amino acid sequence of SEQ ID NO: 1. Through the above results, it was found that the peptide according to an example contributes to skin barrier strengthening and skin anti-aging by increasing skin barrier factors in damaged skin cells.

### Example 6. Confirmation of increased matrix metalloprotease inhibition effect by ultraviolet rays

It has been reported that MMP-1 (matrix metalloproteinase-1) promotes the decomposition of collagen and the reduction of elastin in the dermis layer, thereby creating wrinkles and causing a decrease in elasticity in the skin. Accordingly, in this example, the aim was to confirm the effect of the peptide according to an embodiment on improving extrinsic skin aging, including improving wrinkles and increasing elasticity, etc., by evaluating the change in the expression level of MMP-1 increased by ultraviolet irradiation.

Specifically, NIH3T3 cells or HaCaT cells were seeded in a 6-well plate at a density of 5×10⁵ cells/well and cultured for 24 hours. Next, the cells were washed once with serum-free DMEM media, and 10 µM, 50 µM or 1000 µM of the peptide consisting of the amino acid sequence of SEQ ID NO: 1 was dispensed into 1 mL of the serum-free media, and cultured in a CO₂ incubator at 37 °C for 1 hour. Next, the above culture was transferred to an e-tube, mixed with 1 ml of PBS, and dispensed into a well plate. Next, using a UV irradiator (VILBER LOURMAT, Cat No.: 3102-BSU, France), 6 J/cm² ultraviolet rays were irradiated to NIH3T3 cells, and 20 mJ/cm² ultraviolet rays were irradiated to HaCaT cells. Next, the PBS in the well plate was removed, 900 µL of the culture medium in which the peptides were dispensed was added, and the plate was cultured in a CO₂ incubator at 37 °C for 24 hours. Next, the culture was transferred to an e-tube, washed twice with PBS, and the culture was dissolved by adding a dissolution buffer. Next, the dissolved culture was treated with 5X sample buffer to prepare a sample, and then SDS-PAGE was performed to separate the proteins, which were then transferred to a PVDF membrane. Next, the PVDF membrane was blocked for 1 hour at room temperature using 5 % skim milk, and anti-MMP-1 antibody (Abcam, Cat. No.: ab137332, UK) was diluted 1:500 in 5 % skim milk and reacted with the membrane for 2 hours. Next, after washing three times for 10 minutes each with 0.1 % PBS-T (0.1% Tween-20 in PBS), and goat anti-rabbit IgG (Jackson Immune Research, Cat. No.: 111-035-033, USA) was diluted 1:3000 in 5 % skim milk and reacted with the membrane for 2 hours. Next, MMP-1 was detected using 15. ECL solution (GE Healthcare, Cat. No.: RPN2232, USA). Meanwhile, in this example, an untreated group was used as the control group, a group irradiated with ultraviolet rays only was used as the comparison group, and a group to which Nac was added was used as the positive control group.

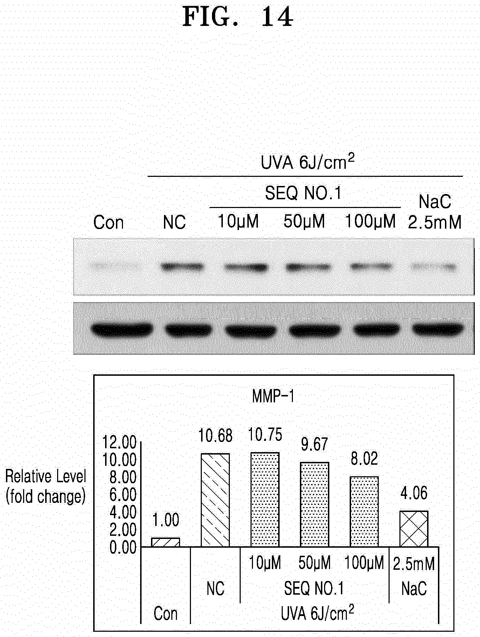

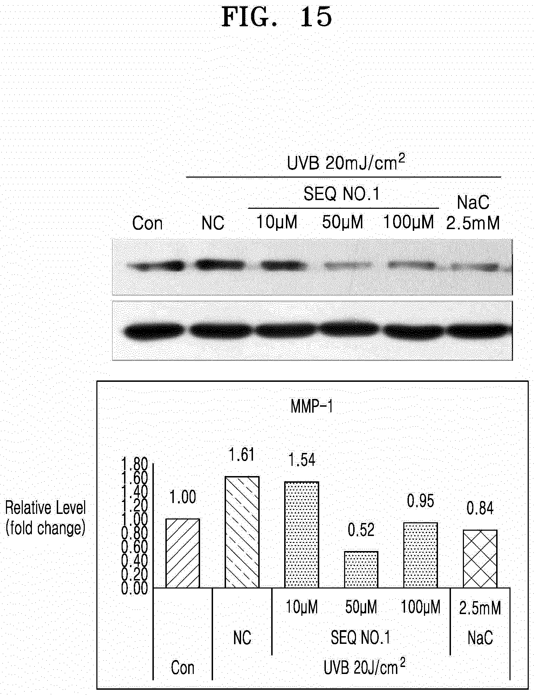

As a result, as shown in FIG. 14 and FIG. 15, it was confirmed that the expression of MMP-1 increased by ultraviolet irradiation was reduced or restored by the peptide consisting of the amino acid sequence of SEQ ID NO: 1. Through the above results, it was found that the peptide according to an embodiment contributes to improving aging such as wrinkle formation and loss of elasticity caused by external factors by regulating the level of collagen decomposition enzyme, etc., in other words, MMP-1.

### Example 7. Confirmation of inhibitory effect on melanin production

B16F10 cells, a mouse melanoma cell line, were seeded at a density of 5×10⁴ cells/well in a 6-well plate and cultured for 16 hours. Next, the culture medium was replaced with a medium including a 2 % serum, and 200 ng/ml of α-MSH (α-melanocyte-stimulating hormones) was added to promote melanin production, in addition, 50 µM or 100 µM of each peptide consisting of the amino acid sequence of SEQ ID NO: 1 was added, and then cultured for 72 hours. Next, the cultured cells were dissolved with 1N NaOH and the absorbance at 450 nm was measured. Meanwhile, an untreated group (Con) was used as the control group, a group to which only α-MSH was added was used as the negative control group, a group to which the peptide consisting of the amino acid sequence of SEQ ID NO: 2 or 3 and α-MSH were added (50 µM, 100 µM) as the comparison group, and a group to which Arbutin (200 µM, 500 µM), known as a skin whitening agent, and α-MSH were added was used as the positive control group.

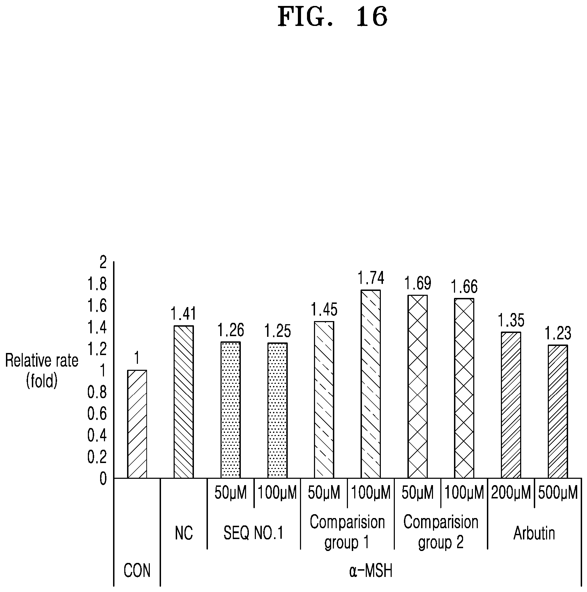

As a result, as shown in Fig. 16, it was confirmed that melanin production increased in the comparison group consisting of a sequence similar to the peptide according to an embodiment, whereas melanin production decreased by the addition of the peptide consisting of the amino acid sequence of SEQ ID NO: 1. Through the above experimental results, it was found that the peptide consisting of the amino acid sequence of SEQ ID NO: 1 according to an embodiment not only has the state of the skin improvement effect described above, but also has a skin whitening effect.

### Formulation Example 1. Preparation of peptide nanosomes

50 mg of the peptide of Example 1 above was dissolved in 500 ml of distilled water by sufficient stirring. The composite solution was mixed with 5 g of lecithin, 0.3 ml of sodium oleate, 50 ml of ethanol, and a small amount of oil, and the total amount was adjusted to 1 L with distilled water, and the mixture was then emulsified using a mat high pressure to produce peptide nanosomes with a size of about 100 nm.

### Formulation Example 2. Softening Lotion

A softening lotion including the peptide according to an example and consisting of the following composition was prepared using a method known in the art.

**[Table 4]**

| **Ingredient** | **Content (wt%)** |
|---|---|
| Peptide | 1.0 |
| 1,3-Butylene glycol | 6 |
| Glycerin | 4 |
| PEG 1500 | 1 |
| Sodium hyaluronate | 1 |
| Polysorbate 20 | 0.5 |
| Ethanol | 8 |
| Benzophenone-9 | 0.05 |
| Preservatives, colorants | As required |
| Fragrance | As required |
| Purified water | Remainder |
| Total | 100 |

### Formulation Example 3. Nutrition Cream

A nutrition cream including the peptide according to an example and consisting of the following composition was prepared using a method known in the art.

**[Table 5]**

| **Ingredient** | **Content (wt%)** |
|---|---|
| Peptide | 2.5 |
| Meadowfoam oil | 3 |
| Cetearyl alcohol | 1.5 |
| Stearic acid | 1.5 |
| Glyceryl stearate | 1.5 |
| Liquid paraffin | 10 |
| Beeswax | 2 |
| Polysorbate 60 | 0.6 |
| Sorbitan sesquioleate | 2.5 |
| Squalane | 3 |
| 1,3-Butylene glycol | 3 |
| Glycerin | 5 |
| Triethanolamine | 0.5 |
| Tocopheryl acetate | 0.5 |
| Preservatives, colorants | As required |
| Fragrance | As required |
| Purified water | Remainder |
| Total | 100 |

### Formulation Example 4. Nutrition Lotion

A nutrition lotion including the peptide according to an example and consisting of the following composition was prepared using a method known in the art.

**[Table 6]**

| **Ingredient** | **Content (wt%)** |
|---|---|
| Peptide | 3.0 |
| 1,3-Butylene glycol | 4 |
| Glycerin | 4 |
| Cetearyl alcohol | 0.8 |
| Glyceryl stearate | 1 |
| Triethanolamine | 0.13 |
| Tocopheryl acetate | 0.3 |
| Liquid paraffin | 5 |
| Squalane | 3 |
| Oils | 2 |
| Polysorbate 60 | 1.5 |
| Sorbitan sesquioleate | 0.5 |
| Carboxyvinyl polymer | 1 |
| Preservatives, colorants | As required |
| Fragrance | As required |
| Purified water | Remainder |
| Total | 100 |

### Formulation Example 5. Essence

An essence including the peptide according to an example and consisting of the following composition was prepared using a method known in the art.

**[Table 7]**

| **Ingredient** | **Content (wt%)** |
|---|---|
| Peptide | 2.0 |
| Glycerin | 10 |
| 1,3-Butylene glycol | 5 |
| PEG 1500 | 2 |
| Allantoin | 0.1 |
| DL-Panthenol | 0.3 |
| EDTA-2Na | 0.02 |
| Hydroxyethyl cellulose | 0.1 |
| Sodium hyaluronate | 8 |
| Carboxyvinyl polymer | 0.2 |
| Triethanolamine | 0.18 |
| Octyldodeceth-16 | 0.4 |
| Ethanol | 6 |
| Preservatives, colorants | As required |
| Fragrance | As required |
| Purified water | Remainder |
| Total | 100 |

The foregoing description of the present disclosure is for illustrative purposes only, and one that has ordinary skill in the art to which the present disclosure belongs will understand that it may be readily adapted to other specific forms without altering the technical ideas or essential features of the present disclosure. Therefore, the examples described above should be understood in all respects as illustrative and not restrictive.

## Claims

1. A cosmetic composition for improving the state of skin, comprising, as an active ingredient, a peptide consisting of an amino acid sequence of SEQ ID NO: 1.

2. The composition of claim 1, wherein an N-terminus of the peptide is bonded to any one protecting group selected from the group consisting of an acetyl group, a fluoreonylmethoxycarbonyl group, a formyl group, a palmitoyl group, a myristyl group, a stearyl group, a butoxycarbonyl group, an allyloxycarbonyl group, and polyethylene glycol (PEG).

3. The composition of claim 1, wherein a C-terminus of the peptide is bonded to any one protecting group selected from the group consisting of an amino group (-NH₂), a tertiary alkyl group, and azide (-NHNH₂).

4. The composition of claim 1, wherein the peptide exhibits any one or more characteristics selected from the group consisting of:
(a) enhancement of expression of an extracellular matrix component, being collagen type 1 (Col1a1), fibronectin, elastin, or hyaluronic acid;
(b) enhancement of expression of a skin barrier factor, being sirtuin-1 (SIRT-1) or aquaporin-3 (AQP3);
(c) reducing reactive oxygen species levels;
(d) reducing expression of matrix metalloproteinase-1 (MMP-1); and
(e) inhibiting melanin production.

5. The composition of claim 1, wherein the peptide is formulated in nanosome form.

6. The composition of claim 1, wherein the improvement of the state of skin is improvement in wrinkles, improvement in skin elasticity, wound healing, strengthening of a skin barrier, inhibition of skin aging, or skin whitening.

7. The composition of claim 6, wherein the skin aging is skin aging caused by ultraviolet rays.

8. A pharmaceutical composition for preventing or treating melanin hyperpigmentation disease, comprising, as an active ingredient, a peptide consisting of an amino acid sequence of SEQ ID NO: 1.

9. The composition of claim 8, wherein the melanin hyperpigmentation disease is melasma, freckles, senile pigmentation, or solar lentigines.
